# EUROPEAN PATENT APPLICATION

(11) **EP 1 153 611 A2**
(43) Date of publication of application: **14.11.2001**
(21) Application number: 01110887.5
(22) Date of filing: 04.05.2001
(51) Int. Cl.: A61K 39/395, A61L 27/22, A61L 31/16, A61P 41/00, A61P 37/00

(54) **Pharmaceutical composition for the treatment of fibrosis**

(30) Priority: 05.05.2000 IL 13599300
(71) Applicant: Omrix Biopharmaceuticals Ltd., Nes-Zionna (IL)
(72) Inventor: Shoenfeld, Yehuda, Ramat Gan (IL); Nur, Israel, 79860 Moshav Timurim (IL)
(74) Representative: VOSSIUS & PARTNER

(57) **Abstract**

The preparation of a pharmaceutical composition comprising as an active ingredient intravenous immunoglobulin for the treatment of fibrous-deposition conditions.

## Description

### FIELD OF THE INVENTION

The present invention concerns a pharmaceutical composition for the treatment of conditions associated with fibrous deposition.

### BACKGROUND OF THE INVENTION

Deposition of collagen, laminin, fibrinogen and other connective molecules is a part of the normal process of inflammation and healing. However, the synthesis of collagen is involved also in a number of pathological conditions all characterized by the formation of fibrous tissue caused by the deposition of abnormally large amounts of collagen. These conditions include disorders associated with primary or secondary fibrosis such as: systemic sclerosis, graft-versus-host disease, myelo fibrosis, pulmonary and hepathic fibrosis (cirrhosis), post operational adhesions and a large variety of autoimmune disorders which are distinguished by excessive production of connective tissue which result in the disruption of normal tissue's architecture and function. In several diseases such as SLE and Sjogren's syndrome the problem does not reside in the disease process itself, but in the myelofibrotic process which leads to fibrosis.

Abnormal deposition of collagen also characterizes such pathological conditions as formation of keloids, which are benign fibronic tumors of the skin; formation of scar-tissue which may be a result of surgical intervention or due to hypertrophic processes; and formation of adhesions between organs of the abdominal or pelvic cavity which is a frequent and undesired complication of abdominal pelvic surgery.

Treatment of conditions characterized by excess fibrous deposition have been attempted by the administration of cytotoxic drugs in order to decrease proliferation of collagen-producing fibroblasts administration of inhibitors of collagen synthesis such as nifedipine, and phenytoin; by administration of inhibitors of collagen cross-linkers, such as β-aminopropionitrile and D-penicillamine.

Today, the most common methodology for treatment of fibrous deposition is administration of corticosteroids. However, said administration may be incomplete often leading to relapse that necessitate adjunct immunosuppressive therapy.

Other currently used methodologies of treatment include: administration of colchicine which slows secretion of collagen into the extracellular matrix (ECM), administration of interferon-γ (IFN-γ), an inhibitor of protein synthesis. Recently halofuginone was also found to be effective as an anti-fibrotic therapy (Pines, M. and Nagler, A., *Gen. Pharmac.,* **30**:445-550, 1998).

Human normal immunoglobulin for intravenous administration (IVIG) is a highly purified IgG type preparation made from pooled human plasma collected from thousands of healthy blood donors. The spectrum of antibody specificity expressed is extremely large, and IVIG recognizes a large number of bacterial, viral and other infectious agents. In addition, these characteristics facilitated the use of IVIG for the combat of various infectious agents in immune deficient individuals. The anti-idiotypic activity of IVIG is utilized for the treatment of various autoimmune diseases, typically as an agent in diseases manifested by pathogenic idiotypic autoimmunity.

IVIG is currently being used as an immunomodulatory agent in autoimmune diseases in allogeneic bone marrow transplantation. IVIG has a well established effect in idiopathic thrombocytopenic purpura (ITP), Kawasaki disease and Guillain-Barre syndrome. Control trials have also shown clinical efficacy of IVIG in immune neutropenia, myasthenia gravis, multifocal motor neuropathy, chronic inflammatory demyelinating polyneuropathy, relapsing-remitting multiple sclerosis, myastesia gravis and refractory dermatomyastosis (Dalakas *et al. Ann. Int. Med.*, 1997, **126**:721-730). IVIG has also been used to treat 50-60 unapproved conditions with beneficial effects in most of them including women with recurrent abortions of unknown causes (Carp *et al., Am. J. Reprod. Immunol.,* 1996, **35**:360-362), heparin induced thrombocytopenia (Winder *et al., J. Clin. Immunol.,* 1998, **18**:330-334), systemic vasculitis (Levy *et al., Int. Arch. Allergy Immunol.,* 1999, **119**:231-238) and systemic sclerosis (Levy *et al., Clin. Rhematol.,* 2000, **19**:200). In addition, a few animal models have shown the beneficial effect of IVIG in both prevention and treatment of experimental anti-phospholipid syndrome (APS) and systemis lupes erythrematosys (SLE) (Krause *et al., J*. *Rhematol.* 1995, **22**:1068-1074), which was also confirmed in humans (lupus) (Levy *et al., Lupus* **8**: 705-712, 1999).

### SUMMARY OF THE INVENTION

The present invention is based on the surprising finding that IVIG is useful in the treatment of six conditions, all of which are characterized by excess deposition of fibrous material (systemic lupus erythrematosys with mylelofibrosis, Sjögren's syndrome, idiopathic mylefibrosis, hepatitis-C-cirrhosis, scleroderma, and autoimmune pulmonary fibrosis).

Thus, the present invention concerns use of intravenous immunoglobulin (IVIG) for the preparation of a medicament for the treatment of fibrous deposition conditions (FDC).

The term *"fibrous deposition condition"* refers to any physiological or pathological condition which can be cured, ameliorated or prevented by decreasing the amount of fibrous material deposited in tissues.

The term *"fibrous"* (also termed at times as *"fibrous material"*) refers to connective tissue having fibrillar element, typically deposition dominated by collagen-rich extracellular matrix. This term may refer to proliferation of cells of the connective tissue as well as to increased deposition of extra-cellular matrix.

The tissues in which said fibrous deposition is manifested can be for example: kidney, heart, lung, liver, skin, uterus, eye, bone marrow and in fact virtually any tissue which contains connective tissue.

The fibrous deposition conditions (FDC) may be due to conditions wherein the fibrous deposition is the cause of a pathologic condition, disease or disorder, or in cases (secondary fibrosis) where the fibrous deposition is the result of said pathologic condition , disease or disorder.

The FDC may be due to genetic causes, due to various non-genetic processes such as infection by microorganisms, toxic substances, irradiation, tumors, inflammation, autoimmune reaction or due to trauma, for example, by surgery, burns, injury and the like.

Examples of conditions which are caused or are a result of abnormally high fibrous depositions are: cirrhosis, idiopathic mylefibrosis associated with systemic lupus erythematosis, Sjögren's syndrome, scleroderma (systemic sclerosis); pulmonary fibrosis, graft vs. host disease, keloid formation, post surgical adhesions, hypertrophic scars, and many others.

The term *"treatment"* in the context of the invention refers to: curing the condition, amelioration of the condition, as well as prevention of the fibrous deposition before it has occurred.

Amelioration or cure of the condition may be determined by several methods as follows:
(i) by measuring decrease in the amount of fibrous deposition as can be carried out for example by measurement of prolyl-hyroxylase activity, attenuation of incorporation of radiolabeled proline into collagenase-digested proteins and by determination of the level of collagen α1 gene expression (especially the lungs);
(ii) where the determination is of a restenosis-related fibrosis, the determination may be carried out by measuring reduction of initial thickening measured by morphometric analysis of the neointima/media ratio;
(iii) by measuring decrease in the size and consistency of scar tissue, for example, as can be achieved by various imaging procedures, by biopsy and histochemical stains;
(iv) by measuring functional capacities of organs such as liver, kidney, bone marrow.

Where a disease or surgical procedure is known to form scar tissue or cause formation of adhesions, it is possible to administer to the patient the pharmaceutical composition of the invention at the outset of the disease or before the surgical procedure takes place in order to avoid future excess fibrous deposition and to avoid scar or adhesion formation.

The term *"intravenous immunoglobulin" (IVIG)* refers, in the context of the present invention, to a highly purified IgG preparation made from pooled human plasma collected from thousands of healthy blood donors.

Although the immunoglobulins are referred as *"intravenous immunoglobulins",* this terminology is used merely for convenience sake since this is a term well known in the art, and by no means is meant to indicate that the composition of the invention can be administered only by intravenous administration. The pharmaceutical compositions of the invention may be administered by intravenous administration, by intraperitoneal administration, by direct injection of the IVIG into lesions, scars or connective tissue as well as by subcutaneous and intramuscular administration.

In accordance with another aspect the present invention concerns a composition of matter comprising IVIG and additional medicinal substances used in such procedures which are known to produce scar tissue as a result of excessive deposition of fibrous tissue. For example, the composition of matter in accordance with this aspect of the invention may be IVIG together with medicinal glue which is routinely used in surgical procedures. Said composition of matter on the one hand features the gluing properties of the medical glue and on the other hand the anti-fibrous deposition properties of IVIG so as to prevent adhesion and scar formation which are many times post-surgery complications.

By yet another aspect the present invention concerns an implant comprising IVIG in a form capable of diffusion from the implant to the surrounding tissue. Many medical procedures include the insertion of a medicinal implant within the body of an individual. Examples of such medicinal implants are: intrauterine devices used as contraceptives; stents used to support blood vessels; screws used in orthopedic devices; insulin pumps; pacemakers implanted in the heart and the like.

Many times an excess deposition of fibrous material is formed around the implanted medicinal device which may hinder the intended activity of the device, and/or disrupt the normal activity of the surrounding tissue. In such a case, it would have been desirable to prevent said excess fibrous deposition, minimize scar and adhesion formation by ensuring that the implant comprises also IVIG in a form which can diffuse to the surrounding tissue thus preventing said scar and adhesion formation. This may be achieved, for example by dipping the implant, prior to insertion in the body in a liquid preparation comprising the IVIG of the invention. Alternatively, this can be achieved by coating the medicinal implant with a biocompatible viscous substance containing IVIG and capable of its sustained release. Alternatively, the implant may contain a reservoir which holds the IVIG, which is separated from the surrounding tissue by a membrane through which the IVIG may slowly penetrate the body.

Typically, the therapeutic dosage of IVIG is 1 to 3 grams/kg of body weight, most preferably 2 grams/kg of body weight (the gram referring to the amount of protein in the IVIG preparation) given as an IV infusion for 5 days every month.

The therapeutic amount is typically set at 2 grams/kg. The total dosage may be administered as a single dose by a 10 hour infusion period or alternatively may be divided into five daily dosage of 400 mg/kg which minimizes the risk of adverse side effects. The rate of diffusion in accordance with the method of the invention usually exceeds 200 mg/hr or 0.08 mg/kg/minute.

In long term therapy, the IVIG infusion is repeated every 4-8 weeks according to the patient's response and objective kinds of disease reoccurrence.

As indicated above, the administration may also be by injection directly to the lesions.

### BRIEF DESCRIPTION OF THE DRAWINGS

In order to understand the invention and to see how it may be carried out in practice, preferred embodiments will now be described, by way of non-limiting example only, with reference to the accompanying drawings, in which:
**Figs. 1A - 1F** are stained skin tissue sections showing the effect of IVIG treatment on collagen expression in Tsk+ mice; and
**Fig. 2** is a graph illustrating TGFβ1, IL-4 and IFNγ secretion by splenocytes derived from Tsk+ mice treated with IVIG.

### DETAILED DESCRIPTION OF THE INVENTION

### I. ANIMAL MODEL

### A. MATERIALS AND METHODS

**(a) Mice and treatment protocol:** Tsk/+ mice were used as an *in-vivo* model for experimental scleroderma-like disease. Three mice were sacrificed at the age of 4 weeks for histological and cytokine analysis at an early stage of disease development The remaining mice were divided into 3 groups:
   A: Ten mice were treated with IVIG.
   B: Ten mice received 200ul of 1% maltose/mouse twice a week.
   C: Ten mice remained untreated.
**(b) IVIG administration:** 2.2mg in 200ul of 2% maltose was administered iv to each 4week old mouse, twice a week over a period of 8 weeks. A total of 35mg IVIG /17gr mouse (equivalent to 2gr/kg) was administered. The mice were opened one week after treatment.
**(c) Collagen analysis of the affected Tsk/+ skin by histology:**
   *Sirius red staining:* Skin samples were fixed with 4% paraformaldehyde, embeded in paraffin blocks and cut into 5µ tissue sections. The samples were deparafinized by xylene and different dilutions of ethanol ( 100% up to 50%). Following incubation in 0.03% Fast green in picric acid (3 min), the samples were exposed to Sirious red (0.1% in picric acid) for 20 min. The slides were dehydrated in ethanol 70% up to 100% and xylene.
   *In-situ hybridization:* Deparaffinized slides underwent prehybridization with SSC solution, proteins were digested with proteinase K (10 mg/ml 10 min at 37C). DIG labeled collagen-I RNA probe was hybridized with the tissue sections overnight at 60°C in 5xSSC. Washed slide were blocked with 3% goat serum in TBS. The collagen mRNA was detected by anti-DIG antibodies conjugated to alkaline phosphatase and exposed to substrate.
**(d) Cytokine analysis:**
   IFNy (Th1), IL-4 (Th2) and TGFβ1 (Th3) secretion by splenocytes *in-vitro* was studied.

### IFNγ production by splenocytes:

Isolated splenocytes (2x10⁶ cells/ml) were cultured *in vitro* in the presence of Con-A 2µg/ml in serum-free media over a period of 48hrs. The culture supernatant was collected and tested for the presence of IFNγ using commercial capture ELISA (DuoSet ELISA kit, R&D systems, DY485). Briefly, ELISA plates were coated with rat anti-mouse IFNγ 4µg/ml and incubated overnight at room temperature. Washed plates were blocked with 1%BSA in 0.05% Tween 20 in TBS for 2hrs at room temperature.

Serial dilutions of standard (recombinant mouse IFNγ) were applied to the plate as well as samples of 100µl of culture fluids in duplicates. Following 2hrs of incubation at room temperature, the presence of IFNγ was probed by biotinylated goat anti-mouse IFNγ 400ng/ml, for 2hrs at room temperature. Streptavidin-HRP (1/200) was added for 20 min at room temperature, and the plates were covered with aluminum foil to avoid exposure to light. Substrate solution was added for an additional 20min (Tetramethylbenzidine, H₂O₂) and stopped with 50µl 2N H₂SO₄. Between each step the plates were washed 3 times with PBS pH 7.4/0.05% Tween 20. The plates were read at an OD of 450 nm.

### IL-4 production by splenocytes:

Isolated splenocytes (2x10⁶ cells/ml) were cultured *in vitro* in the presence of Con-A 2µg/ml in serum free media during 48hrs. The culture supernatant was collected and tested for the presence of IL-4 using commercial capture ELISA (DuoSet ELISA kit, R&D systems, DY404). Briefly, ELISA plates were coated with rat anti-mouse IL-4 4µg/ml and incubated overnight at room temperature. Washed plates were blocked with 1%BSA in 0.05%Tween 20 in TBS for 2hrs at room temperature.

Different dilutions of standard (recombinant mouse IL-4) were applied to the plate as well as samples of 100µl of culture fluids in duplicates. Following 2hrs of incubation at room temperature, the presence of IL-4 was probed by biotinylated goat anti-mouse 400ng/ml, 2hrs at room temperature. Streptavidin-HRP (1/200) was added for 20min at room temperature, plates were covered with aluminum foil to avoid exposure to light. Substrate solution was added for an additional 20min (Tetramethylbenzidine, H₂O₂) and stopped with 50µl 2N H₂SO₄. Between each step the plates were washed 3 times with PBS pH7.2/0.05% Tween 20. The plates were read at OD of 450 nm.

### TGFβ1 production by splenocytes:

Isolated splenocytes (2x10⁶ cells/ml) were cultured *in vitro* in the presence of Con-A 2µg/ml in serum free media during 72hrs. The culture supernatant was collected and tested for the presence of IL-4 using commercial capture ELISA (DuoSet ELISA kit, R&D systems, DY404). Briefly, ELISA plates were coated with rat anti-mouse TGFβ1 2µg/ml and incubated overnight at room temperature. Washed plates were blocked with 1%BSA in 0.05%Tween 20 in TBS for 2hrs at room temperature.

Different dilutions of standard (recombinant human TGFβ1 in dilution buffer containing 1.4% delipidated BSA) were applied to the plate as well as samples of 100µl of culture fluids (after TGFβ1 activation), in duplicates, 2hrs of incubation at room temperature. The presence of TGFβ1 was probed by biotinylated chicken-anti-human TGFβ1 300ng/ml in dilution buffer (1.4% delipidated BSA), 2hrs at room temperature. Streptavidin-HRP (1/200) was added for 20min at room temperature, plates were covered with aluminum foil to avoid exposure to light. Substrate solution was added for an additional 20min (Tetramethylbenzidine, H₂O₂) and stopped with 50µl 2N H₂SO₄. Between each step the plates were washed 3 times with PBS pH7.2/0.05% Tween 20.The plates were read at OD of 450 nm.

### TGFβ1 activation in the tested samples:

Culture supernatants containing the tested TGFβ1 0.5ml/sample were activated by adding 0.1ml IN HCL, mixing, and incubating 10min at room temperature. The samples were neutralized in the presence of 0.1ml 1.2N NaOH/0.5M HEPES.

### B. RESULTS

### Example I

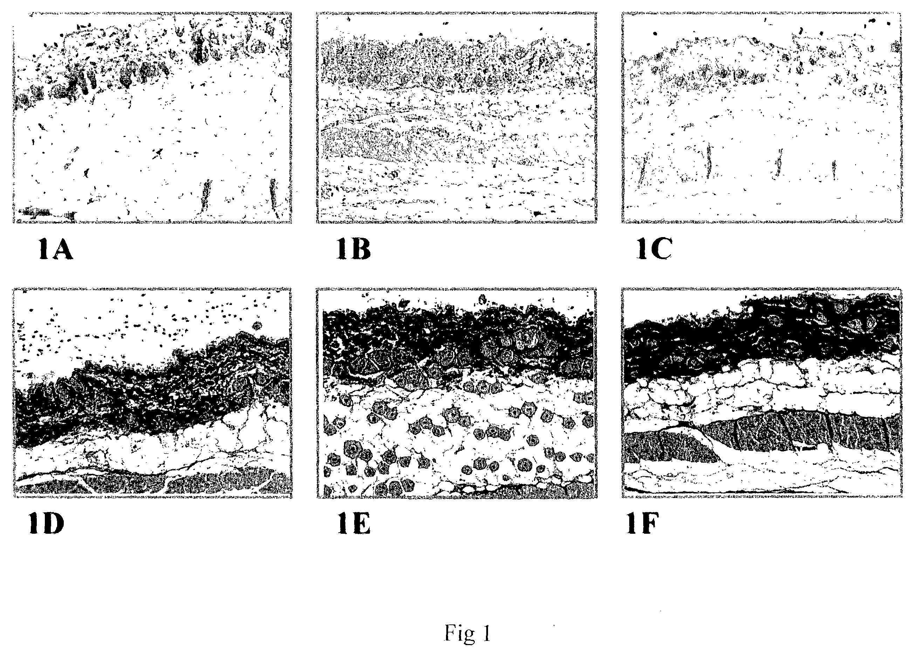

IVIG was administered to 4 month old Tsk+ mice to assess the consequence of IVIG administration on the development of skin fibrosis in an *in-vivo* model of scleroderma-like disease. The result of the IVIG effect was depicted by collagen deposition in the affected mice via collagen staining and collagen-I mRNA expression. As shown in Fig. 1, significant expression of collagen mRNA can be observed in IVIG treated mice (Fig. 1A) in comparison to mice treated with maltose (Fig. 1B) and non treated mice (Fig. 1C). Furthermore, digested collagen in the skin of the IVIG treated mice was documented (Fig. 1D), while significant strong collagen deposits can be observed in the skin of mice treated with maltose or non treated mice (Figs. 1E and 1F, respectively).

### Example II

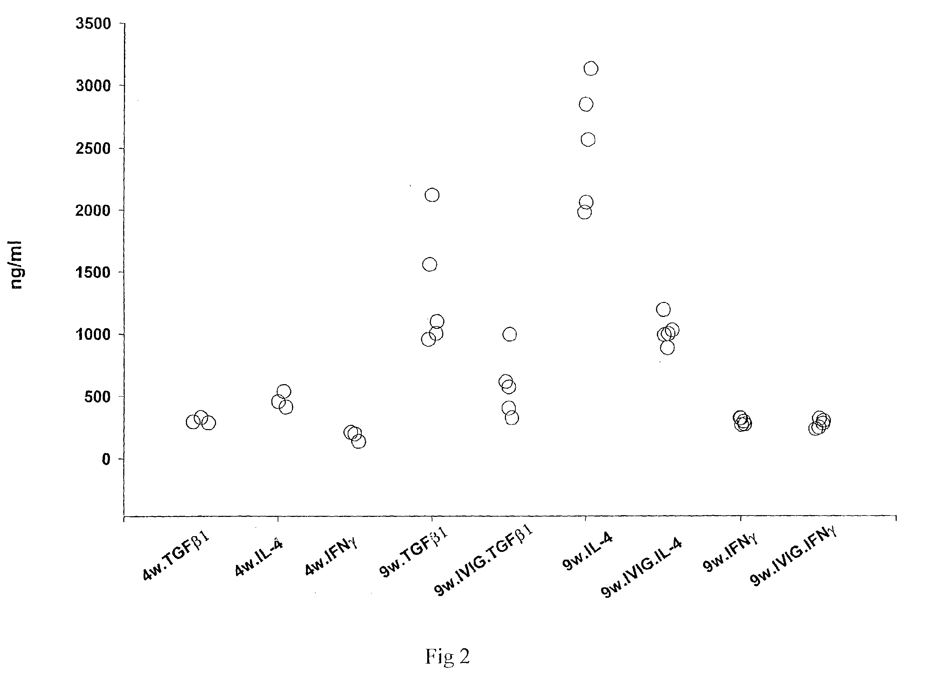

The results summarized in Fig. 2 show inhibition of IL-4 and TGFβ1 secretion by splenocytes derived from the Tsk+ IVIG treated mice as compared to Tsk+ non treated mice (p<0.05). No effect of IVIG was documented on IFNγ secretion (p>0.05).

### II. CLINICAL EXAMPLES

### A. METHODS

Eight patients with different medical conditions in which fibrosis deposition played a significant role were chosen as follows:
1. A patient with systemic lupus erythematosus with myelofibrosis;
2. A patient with Sjögren's syndrome with myelofibrosis;
3. A patient with idiopathic myelofibrosis;
4. A patient with hepatitis-C cirrhosis;
5-7. Three patients with scleroderma(systemic sclerosis); and
8. A patient with autoimmune thrombocytopenia with cutaneous fibrosis.

All eight patients were treated with intravenous gammaglobulin administration IVIG by Isiven (Italy) at a dosage of 2 gram/kg body for 5 consecutive days. The treatment was repeated 1 to 6 times with intervals between the treatment being of about one month.

### B. RESULTS

In all eight patients, regression of the fibrotic process was noticed as determined by the method specified in the publication of Levy *et al*., *Clin. Rhematoid.* 2000, **19**:207, leading in several cases to complete reversion of the disease.

The reversion of the disease was determined in the myelofibrosis cases (systemic lupus erythematosus and Sjögren's syndrome, idiopathic myelofibrosis), by bone marrow biopsy showing a decrease in fibrosis and regeneration of blood proginator cells (corpuscles ); in systemic cirrhosis (scleroderma) the reversion of the disease was determined by improvement in parameters of the skin thickness and elasticity; in hepatitic-C-cirrhosis the improvement was determined by biopsy and collagen staining and by liver function determination.

## Claims

1. Use of IVIG for the preparation of a medicament for the treatment of fibrous deposition conditions.

2. Use according to Claim 1, wherein said FDC is selected from the group consisting of systemic lupus erythrematosys with mylelofibrosis, Sjögren's syndrome, idiopathic mylefibrosis, hepatitis-C-cirrhosis, scleroderma, and autoimmune pulmonary fibrosis.

3. Use according to either of Claims 1 or 2, wherein said medicament is administered by a method of administration selected from the group consisting of intravenous administration, intraperitoneal administration, and intralesional administration.

4. A composition of matter for use in surgery for the prevention of post surgery adhesion comprising IVIG and a medicinal glue.

5. A medicinal implant comprising IVIG, the IVIG being in a form capable of diffusion for the implant to the surrounding tissue.

6. Use of a composition of matter comprising a mixture of IVIG and a medicinal glue for the preparation of a medicament for the prevention of post surgery adhesion.
